# EUROPEAN PATENT SPECIFICATION

(11) **EP 2 476 425 B1**
(45) Date of publication and mention of the grant of the patent: **19.03.2014**
(21) Application number: 11305044.7
(22) Date of filing: 14.01.2011
(51) Int. Cl.: A61K 36/15, A61P 27/02

(54) **Composition comprising OPC and Omega-3 for preventing and/or inhibiting the development of diabetic retinopathy**
Zusammensetzung mit OPC und Omega-3 zur Vorbeugung und/oder Hemmung der Entwicklung von diabetischer Retinopathie
Composition comprenant de l'OPC et de l'oméga 3 pour la prévention et/ou l'inhibition du développement de la rétinopathie diabétique

(43) Date of publication of application: 18.07.2012
(73) Proprietor: Visiotact Pharma, 78180 Montigny-le-Bretonneux (FR)
(72) Inventor: Hadj-Slimane, Reda, 75015, Paris (FR); Hadj-Slimane, Tewfik, 48000 Relizane (DZ); Lepelletier, Yves, 91200 Athis-Mons (FR)
(74) Representative: Icosa

(56) References cited:
- WO-A2-02/47493

## Description

### FIELD OF INVENTION

The present invention relates to a composition for improving eye health, and particularly to reduce the risk of eye damage associated with diabetes, such as, for example, diabetic retinopathy. The present invention thus relates more specifically to a composition for promoting or maintaining visual health, preventing and/or inhibiting the development of eye conditions or disorders associated with diabetes.

### BACKGROUND OF INVENTION

Diabetes or diabetes mellitus is a group of chronic metabolic diseases characterized by a high blood sugar, either because the body fails to produce insulin (which is the case in type-1 diabetes) or because cells are resistant to insulin, and do not respond to the hormone (type-2 diabetes). With the increase of the obesity rate (among other factors) the number of diabetic patients, more specifically of patients affected by type-2 diabetes, increases dramatically. Today, some medical studies value that 7.8% of the European population and 7.9% of the American population suffer from type-2 diabetes. Moreover, about 8.2% of the world population has an impaired glucose tolerance (IGT), a pre-diabetic state that may precede type-2 diabetes by many years. Diabetes, when present in the body over many years, can give rise to all sorts of complications. These include heart disease, kidney disease, neuropathy and eye conditions. Diabetes can affect the eye in a number of ways: cataract, glaucoma, molecular oedema and diabetic retinopathy are considered herein as possible diabetes-related eye conditions.

### Diabetic retinopathy

Diabetic retinopathy (DR) is the result of microvascular retinal changes: with chronic hyperglycemia, blood vessels may be injured and fail to bring nutrients and oxygen to retinal cells. Moreover, hyperglycemia-induced intramural pericyte death and thickening of the basement membrane lead to incompetence of the vascular walls, and may lead to the obstruction of blood capillaries. These damages also change the formation of the blood-retinal barrier and make the retinal blood vessels become more permeable. Two phenomena are observed: the obstruction of blood capillaries may lead to retinal ischemia, and the hyper-permeability of retinal blood vessels may lead to retinal edema. Two types of DR may be distinguished: (i) non proliferative DR is characterized by the permeability of small blood vessels, leading to the accumulation of liquid and exudates at the level of the retina. When blood is accumulating in the macula (the central zone of the retina), a macular edema may form, leading to a progressive vision loss and possibly to blindness; (ii) proliferative DR results from the occlusion of small capillaries: the lack of oxygen in the retina causes fragile, new blood vessels to grow along the retina and in the vitreous humor. These new blood vessels may bleed, cloud vision, and destroy the retina. Fibrovascular proliferation may also cause tractional retinal detachment. Intraretinal hemorrhage or retinal detachment may lead to sudden and irreversible vision loss. Diabetic retinopathy is the first cause of blindness before the age of 60. All people with diabetes (either type 1 or type 2) present a risk of developing DR: estimation is that about 40% of the diabetic population have a DR. After 20 years of diabetes, more than 90% of patients with Type 1 diabetes have a DR, and about 40% have a proliferative DR. In Type 2 diabetes, more than 20% of newly diagnosed patient already have a DR. In this form of diabetes, the major risk is macular edema. After 15 years of diabetes, 2% of diabetics are blind and 10% suffer from a visual impairment. Three treatments exist for slowing or stopping further vision loss resulting from DR. These treatments are :
- laser photocoagulation (coagulation of the blood vessels of the retina, this treatment is indicated for the treatment of proliferative retinopathy),
- treatment with laser of diabetic maculopathy (used if there is a prolonged vision loss) and
- vitrectomy (indicated for patients with proliferative DR with retinal detachment).

Each of these treatments are very invasive, and anyway, they do not cure DR.

### Macular oedema

The most common complication causing vision loss associated with diabetes in patients with non-proliferative retinopathy is macular oedema. This pathology is a macular swelling appearing when retina small blood vessels are altered "ie dilated" inducing generally leaky and fluid builds up. It is the most common cause of visual impairment. Loss of vision can occur swiftly and treatment is not as successful.

### Glaucoma

Another diabetes-related eye condition is glaucoma. Glaucoma is an eye disorder characterized by extremely high pressure in the eyeball and causes the optic disk to be damaged. Although the symptoms might not show up until it is in the advanced stages and is irreversible. This causes the optic disk to be damaged. People should get regular eye exams especially after the age of 40.

In the later stages of Glaucoma there is a loss of peripheral or side vision capability. Blurred vision, blind spots, and halos around lights may occur. Poor night vision is also a late stage symptom of Glaucoma. Left untreated blindness could occur.

The intraocular pressure caused by glaucoma damages nerves and vessels in the eye, resulting in changes in vision.

The subjects developing an eye disorder related with diabetes may be aware of their evoluting condition. In some patient, a mild form of retinopathy or glaucoma may progress to a sight threatening condition. However, in the knowledge of the Applicant, there is no efficient composition meeting the needs of this subject, i.e. preventing or inhibiting the development of the diabete-related eye-condition. By searching the prior art, the Applicant found EP1 214 893, which describes compositions for promoting health, especially in patients diagnosed with diabetes, and comprising several ingredients such as vitamins, unsaturated fatty acids, carotenes, minerals and oligoproanthocyanidins (OPC). Also, US2010/0021533 describes compositions for improving a person's well being, and comprising vegetal extracts, vitamins, minerals, fatty acids and Pine Tree extracts (an extract comprising OPC). Pine Tree extracts are defined as components, which provide significant health protection in diabetes and cause prevention and an improvement of diabetic retinopathy.

However, none of these patent applications give an indication, such as scientific evidences, that the described compositions are beneficial against diabetes-related eye disorders or conditions.

To the Applicant's knowledge, three major pathways have to be controlled in order to prevent or to slow down the development of diabetes-related eye conditions. First is glycemia, as chronic hyperglycemia may lead to retinal ischemia or intraocular pressure. Second are inflammatory processes, involved in the early phases of the disease. Third is glycation, leading to the production of AGE (advanced glycation end products), which may induce micro-vascular and macro-vascular lesions. AGE may contribute to the initiation and progression of diabetic retinopathy (Stitt et al., Pharmacological Reports, 2005, 57, suppl. 156-158).

Surprisingly, the Inventors herein demonstrate a synergic effect of OPC and unsaturated fatty acids, especially omega-3 fatty acids, on the control of these three pathways. Particularly, the combination of these ingredients in the composition of the invention leads to the alleviation of several ocular symptoms of diabetes-related conditions.

### SUMMARY OF INVENTION

Consequently, this invention relates to a composition comprising oligoproanthocyanidins (OPC) and omega-3 fatty acids, for use in preventing a diabetes-related eye condition in a subject or stabilizing the development and/or the progression of a diabetes-related eye condition in a subject.

The composition of the invention further comprises Vitamin B1, Vitamin B6, chromium and magnesium.

According to the invention, the OPC may natural, synthetic or hemisynthetic. In a preferred embodiment, the OPC is natural and the composition comprises as OPC a OPC-containing natural extract, preferably a vegetal extract, more preferably an extract from a plant of the Pinacae family, even more preferably an extract from *Pinus pinaster* or *Pinus maritima.* the source of OPC is a OPC-containing natural extract, preferably an OPC-containing vegetal extract, more preferably an extract from a plant of the Pinacae family, even more preferably an extract from *Pinus pinaster* or *Pinus maritima.*

In a preferred embodiment, the omega-3 fatty acid is DHA, EPA or mixtures thereof, more preferably DHA. In an embodiment, the source of omega-3 fatty acid is natural. advantageously , the omega-3 fatty acid is provided in the composition through a DHA-containing fish oil, preferably the composition comprising a DHA-containing fish oil having both the Epax^{®} and the Qualitysilver^{®} labels.

In an embodiment, the vitamin B1 carried out in the composition of the invention is thiamin or benfotiamin, preferably thiamin.

In an embodiment, the vitamin B6 used in the composition of the invention vitamin B6 is pyridoxamine, pyridoxal, pyridoxine or mixtures thereof, preferably pyridoxamine.

In an embodiment, chromium is present in the composition of the invention in the form of chromium picolinate, chromium chloride, chromium GTF^{®}, chromium polynicotinate or mixtures thereof, preferably chromium picolinate.

In an embodiment magnesium is present in the composition of the invention in the form of magnesium gluconate, magnesium citrate, magnesium chloride, magnesium malate, magnesium orotate, sea magnesium, preferably magnesium gluconate.

In an embodiment, the composition is for use in diabetes-related eye conditions, which are conditions of the interior of the eye, preferably glaucoma, edema or retinal conditions, especially diabetic retinopathy.

This invention also relates to a functional food, a nutraceutical composition or a food or dietary supplement comprising a composition of the invention.

This invention also relates to a medicament comprising the composition of the invention.

This invention also relates to a pharmaceutical composition comprising the composition of the invention, in association with any pharmaceutically acceptable excipient or vehicle. Advantageously, the composition is a form suitable for being administered orally, topically, intraocularly or systemically. In an embodiment, the pharmaceutical composition of the invention is for use in treating, preventing or stabilizing a diabetes-related eye condition, disease or disorder of the eye, comprising administering to a subject in need thereof a therapeutic amount of the composition of the invention.

In an embodiment, the condition, disease or disorder of the eye is diabetic retinopathy or glaucoma. In an embodiment, the subject is a patient diagnosed with diabetes, or having family antecedent related to diabetes.

This invention also relates to a veterinarian product comprising the composition or functional food, nutraceutical composition or food or dietary supplement of the invention. In an embodiment, the veterinarian product of the invention is for use in a mammal, such as, for example, cat, dog, hamster, rabbit, mouse, gerbil, rat, Guinea pig.

### DEFINITIONS

In the present invention, the following terms have the following meanings:
**"Nutraceutical"**: a product isolated or purified from comestibles. A nutraceutical is demonstrated to have a physiological benefit or provide protection against physiological disorder or discomfort.
"**About"**: preceding a figure means plus or less 10% of the value of said figure.
**"Essential fatty acids"**: fatty acids that humans and other animals must ingest because the body requires them for good health but cannot manufacture them. The same way, an **"essential vitamin"** is a vitamin that humans and other animals must ingest because the body requires them for good health but cannot manufacture nor store them.
**"Dietary supplement",** also known as "nutritional supplement" or "food supplement": a product that contains a vitamin, mineral, herb or other botanical, amino acid, concentrate, metabolite, constituent, extract, or combinations of these ingredients. **"Oral administration":** the administration of a product in the mouth cavity followed by the swallowing of the product. In this case, the substance reaches the systemic circulation through a digestive absorption.
**"Sustained-release kinetic":** describes the slow release kinetic of a compound, over an extended period of time, preferably 1 to 24 hours, more preferably 2 to 12 hours.

### DETAILED DESCRIPTION

The present invention relates to a composition comprising oligoproanthocyanidins (OPC) and unsaturated fatty acids.

### OPC

OPC are flavonoids from the family of leucoanthocyanidins present in a wide range of plants. OPC are organic clusters of dimers (Procyanidols type B-1, B-2, B-3, B-4, B-5, B-6, B-7, B-8; Prodelphinidols; Proanthocyanidols type A), trimers (type C-1, C-2), tetramers and pentamers of proanthocyanidol (poly-épicatéchols, procyanidol-prodelphinidol).

OPC possess anti-allergic and anti-inflammatory properties via the inhibition of cyclooxygenase and lipooxygenase pathways (Canali et al., Int Immunopharmacol, 9 (2009):1145-1149). Moreover, OPC enhance the physical resistance of capillaries (Schönlau & Rohdewald, Int Ophthalmol, 24:161-171, 2002).

Clinical trials have demonstrated that the administration of Pine Tree extracts (extract from *Pinus maritime* or from *Pinus pinaster*) may be useful to decrease the symptoms of diabetes complications and particularly retinal edema in early phases of DR and to enhance microcirculation and visual acuity (Cesarone et al., Angiology, 57(4) 2006:431-436; Steigerwalt et al., J Ocul Pharmacol Ther, 2009, 25(6):537-40).

According to an embodiment, the composition of the invention comprises a natural, synthetic or hemisynthetic extract containing OPC. Advantageously, the natural extract containing OPC is a vegetal extract or a mix of vegetal extracts.

According to an embodiment of the invention, said natural extract containing OPC is selected from the group comprising, but not limited to, the Vitaceae family, the Pinacae family, cocoa, tea, hawthorn, *crataegus sinaica,* elm cortex, red-hulled rice Brown soybean seed, blueberries, Oregon caneberries, *Uncaria sinensis* (China), *Ecdysanthera utilis* (China), *Eriobotrya japonica* (Japan), *Erythrophleum suaveolens* (Africa), *Stryphnodendron adstringens (Brazil), Photinia melanocarpa, Phaseolus vulgaris, Corylus avellana, Fragaria x ananassa, Ribes nigrum, Malus pumila, Juglans regia, Ribes rubrum, Hamamelis virginiana, Crataegus monogyna, Crataegus laevigata* and *Accinum macrocarpon.*

The Vitaceae family comprises *Vitis vinifera* (Vine, grape vine, grape,grape seed) ; *Acareosperma, Ampelocissus, Ampelopsis* (pepper-vine), *Cayratia, Cissus* (treebind, treebine), *Clematicissus, Cyphostemma, Leea, Nothocissus, Parthenocissus, Pterisanthes, Pterocissus, Rhoicissus, Tetrastigma* and *Yua.*

The Pinacae familly comprises *Pinus pinaster* (pine bark extract), *Pinus mesogeensis, Pinus maritima,* Subfamily *Pinoideae Pinus -* pines (about 115 species), Subfamily *Piceoideae Picea -* spruces (about 35 species), Subfamily *Laricoideae Cathaya* (one species), *Larix -* larches (about 14 species), *Pseudotsuga -* douglas-firs (five species), Subfamily *Abietoideae Pseudolarix -* golden larch (one species), *Abies -* firs (about 50 species), *Cedrus -* cedars (two to four species), *Keteleeria* (three species), *Nothotsuga* (one specie) and *Tsuga -* hemlock (nine species).

Examples of extracts containing Procyanidols type B-2 are *Cinchona pubescens* (Chinchona), *Cinnamomum verum* (Ceylon cinnamon), *Crataegus monogyna* (Common hawthorn), *Uncaria guianensis* (Cat's claw), *Vitis vinifera* (Common grape vine), *Litchi chinensis* (litchi), apple and *Ecdysanthera utilis.*

Examples of extracts containing Proanthocyanidin A1 are *Rhododendron spiciferum, Dioclea lasiophylla,* peanut and *Ecdysanthera utilis.*

Examples of extracts containing Proanthocyanidin A2 are horse chestnut (*Aesculus hippocastanum*)*,* cranberry, peanut, *Cinchona Cinchona, Cinchona pubescens, Cinnamomum verum* (Ceylon cinnamon), *Persea americana* (avocado or alligator pear), *Urvillea ulmaceae,* and *Ecdysanthera utilis.*

According to a preferred embodiment, the natural extract containing OPC is an extract of a plant from the Pinacae family, more preferably is an extract of *Pinus pinaster* or of *Pinus maritima.*

Extracts of *Pinus pinaster* are commercially available, such as for example Pycnogenol® from Horphag Research, which is protected by three US patents (4,698,360, 5,720,956 and 6,372,266, which are incorporated herein by reference), or PinePure™ which is purchased from Naturex.

According to an embodiment, the natural extract containing OPC is titrated from 50 to 99% in OPC, preferably from 60 to 90% in OPC, more preferably from 70 to 80% in OPC, more preferably at about 75% in OPC.

According to an embodiment, the composition of the invention comprises an amount of OPC ranging from 1 to 1000 mg, preferably from 5 to 500 mg; in an embodiment, the composition comprises more preferably from about 10 to 112 mg, even more preferably an amount of OPC of about 13 mg; in another embodiment, the composition comprises more preferably 60 to 300 mg of OPC.

According to another embodiment, the composition of the invention comprises a natural extract containing OPC in an amount ranging from 1 to 1000 mg, preferably 1 to 500 mg, more preferably 1.5 to 300 mg. In an embodiment, the composition includes from 7.5 to 215 mg, more preferably from about 20 to 160 mg, even more preferably an amount of natural extract containing OPC of about 20 mg; in another embodiment, the composition comprises more preferably 60 to 300 mg of natural extract containing OPC

### Unsaturated fatty acids

According to the invention, the composition comprises, in combination with OPC, unsaturated fatty acids, preferably poly-unsaturated fatty acids, more preferably omega-3 fatty acids. Omega-3 fatty acids are essential fatty acids mainly present in products from the sea, such as, for example, seafood and fatty fish. Scientific studies have demonstrated that intake of omega-3 fatty acids protects the eye against the development and the progression of pathological retinal angiogenesis (Connor et al., Nat Med, 2007, 13(7):868-873).

According to an embodiment, the composition comprises omega-3 fatty acids, preferably selected from the group comprising linolenic acid (ALA), eicosapentaenoic acid (EPA), docosahexaneoic acid (DHA) and mixtures thereof. Advantageously, the composition of the invention comprises DHA only or DHA and EPA.

DHA is an omega-3 fatty acid naturally present in the retina at the level of the photoreceptor discs, where it is involved in neurotransmission, in rhodopsin activation and in the development of cones and rods. Moreover, DHA has an anti-inflammatory role, inhibits the development of new blood vessels, and extends the lifetime of photoreceptors (SanGiovanni & Chew, Prog Retin Eye Res, 2005, 24(1):87-138). DHA intake may inhibit the inflammatory processes related to DR, as well as angiogenic events associated with the disease (Connor et al., Nat Med, 2007, 13(7):868-73).

According to an embodiment, the composition of the invention comprises an oil fish containing omega-3 fatty acids, preferably containing DHA. Advantageously, the oil fish containing DHA is titrated in DHA from 50 to 100%, preferably 55-85%.

DHA is subject to lipid peroxidation, a natural process corresponding to oxidative degradation of lipids and occurring when an unsaturated lipid is in presence of oxygen or of light. To counteract this degradation process and in order to preserve beneficial effects of DHA, in an embodiment of the invention, the oil fish comprised in the composition is stabilized with the Qualitysilver® process, developed by Polaris (France). The Qualitysilver® process allows the stabilization of rich in polyunsaturated fatty acids against oxidation. This process combines the destruction of lipoxygenases present in the oil to the addition of antioxidant compounds in order to protect the nutritional and organoleptic qualities of the oil.

Moreover, due to their lipophilic nature, products from the sea absorb some pollutants, such as, for example, dioxin and heavy metals. In order to prevent the presence of such pollutants, in an embodiment, the composition of the invention comprises an oil fish treated with the Epax® purification technology developed by Epax AS (Norway).

Fish oil containing DHA is commercially available, for example from the Polaris Company, France. Advantageously, the fish oil containing DHA has both the Epax® and the Qualitysilver® labels.

According to an embodiment, the composition of the invention comprises an amount of DHA ranging from 1 to 10000 mg, preferably from 10 to 2000. In an embodiment, the composition of the invention comprises an amount of DHA ranging from more preferably 50 to 500 mg more preferably about 100 to 125 mg.

According to another embodiment, the composition of the invention comprises DHA through a fish oil containing DHA, said fish oil being present in an amount ranging from 1 to 10000 mg, preferably from 10 to 2000. In an embodiment, the DHA-containing fish oil is present in the composition of the invention in an amount of 50 to 500 mg, more preferably from about 100 to 300 mg, even more preferably an amount of a fish oil containing DHA of about 100 mg.

### Vitamins

According to an embodiment of the invention, the composition further comprises vitamins, preferably a vitamin from group B, more preferably a vitamin selected from the group comprising vitamin B1, vitamin B6, derivatives and mixtures thereof.

Vitamin B1, or thiamin or aneurin, is an essential water-soluble vitamin involved in the metabolism of glucose. The intake of high doses of vitamin B1 may protect diabetic persons against the development of vascular complications. Moreover, Benfotiamin, a lipid-soluble derivative of vitamin B1, may prevent DR by inhibiting three pathways: hexosamine pathway, AGE synthesis and Diacylglycerol (DAG) - protein kinase C (PKC) pathway (Hammes et al., Nat Med, 2003, 9(3):294-299).

According to an embodiment of the invention, the composition comprises vitamin B1 or benfotiamin, preferably vitamin B1 in the form of thiamine hydrochloride, thiamine monohydrate, thiamine monophosphate, thiamine nitrate, more preferably tiamine monophosphate 98% or thiamine monohydrate.

According to an embodiment, the composition of the invention comprises an amount of Vitamin B1 or benfotiamin ranging from 0.1 to 500 mg, preferably from 15 to 250 mg. In an embodiment, the composition of the invention comprises an amount of Vitamin B1 or benfotiamin ranging from 0.5 to 5 mg, more preferably from about 1.1 to 2.2 mg, even more preferably of about 1.1 mg.

Vitamin B6 is an essential water-soluble vitamin. There exist three forms of vitamin B6: pyridoxamine, pyridoxal and pyridoxine. All three forms are precursors of an activated compound known as pyridoxal 5'-phosphate (PLP), which plays a vital role as the co-factor of a large number of essential enzymes in the human body. In diabetes, Vitamin B6 may play a role by inhibiting the formation of AGE (Stitt et al., Diabetes, 2002, 51:2826-32).

According to an embodiment of the invention, the composition comprises pyridoxamine, pyridoxal, pyridoxine or mixtures thereof, preferably, the composition comprises pyridoxamine.

According to an embodiment, the composition of the invention comprises an amount of Vitamin B6 ranging from 10 to 500 mg . In an embodiment, the composition of the invention comprises an amount of Vitamin B6 ranging from 0.1 to 10 mg, preferably from 0.5 to 5 mg, more preferably from about 1.4 to 2.8 mg, even more preferably of about 1.4 mg.

### Inorganic compounds

According to an embodiment of the invention, the composition further comprises inorganic compounds, preferably mineral compounds, more preferably a mineral compound selected from the group comprising chromium and magnesium.

Chromium is a trace element indispensable for human health. Chromium insufficiency may lead to a decrease of blood insulin levels and to an increase of blood glucose as well as to an increase of the risk to develop diabetes (Anderson, Nutr Res Rev, 2003, 16(2):267-75; Cefalu & Hu, Diabetes Care, 2004, 27(11):2741-51).

According to an embodiment, chromium is present in the composition of the invention in the form of chromium picolinate, chromium hydrochloride, chromium chloride hexahydrate, chromium GTF®, chromium polynicotinate, and mixtures thereof. Preferably, the composition comprises chromium picolinate, as this form of chromium is efficiently absorbed and does not show any toxicity (Gargas et al., Drug Metab Dispos, 1994, 22(4):522-9).

According to an embodiment, the composition of the invention comprises an amount of chromium ranging from 5 to 500 µg, more preferably from 10 to 250 µg. In an embodiment, the composition of the invention comprises an amount of chromium ranging from 15 to 150 µg, even more preferably of about 20 µg.

Magnesium is a trace element indispensable for human health and for general function of the organism.

According to an embodiment, magnesium is present in the composition of the invention in the form of magnesium gluconate, magnesium citrate, magnesium chloride, magnesium pyruvate, magnesium bromide, magnesium hydroxyde, magnesium iodide, magnesium lactate, magnesium oxalate, magnesium succinate, magnesium sulfate, magnesium oxide, magnesium malate, magnesium orotate, sea magnesium from the sea, such as for example dead sea or red sea magnesium and mixtures thereof. According to a preferred embodiment, magnesium is present in the composition of the invention in the form of magnesium gluconate, magnesium citrate, magnesium chloride and mixtures thereof. According to a more preferred embodiment, magnesium is present in the composition of the invention in the form of magnesium gluconate. According to an embodiment, the composition of the invention comprises an amount of magnesium ranging from 10 to 1000 mg, preferably from 50 to 500 mg, more preferably from 90 to 350 mg, even more preferably of about 93.75 mg.

According to an embodiment of the invention, the composition further comprises at least one ingredient selected from vitamins and inorganic compounds, wherein vitamins are selected from vitamins of B group, preferably from the group comprising vitamin B1, vitamin B6 and mixtures thereof, and wherein inorganic compounds are selected from minerals, preferably from the group comprising chromium and magnesium.

According to an embodiment of the invention, the composition further comprises at least one ingredient selected from the group comprising vitamin B1, vitamin B6, chromium and magnesium.

According to an embodiment of the invention, the composition further comprises vitamin B1, vitamin B6, chromium and magnesium.

According to an embodiment of the invention, the composition comprises OPC; unsaturated fatty acids, preferably omega-3 fatty acids, more preferably DHA; vitamin B1; vitamin B6; chromium and magnesium.

According to an embodiment of the invention, the composition consists of OPC; unsaturated fatty acids, preferably omega-3 fatty acids, more preferably DHA; vitamin B1; vitamin B6; chromium and magnesium.

According to a preferred embodiment, the composition comprises or consists of OPC, DHA, vitamin B1, vitamin B6, chromium and magnesium.

According to an embodiment, the composition comprises or consists of:
- an amount of OPC ranging from 1 to 200 mg, preferably from 5 to 150 mg, more preferably from about 14 to 112 mg, even more preferably an amount of OPC of about 13 mg; or a natural extract containing OPC in an amount ranging from 1.5 to 300 mg, preferably from 7.5 to 215 mg, more preferably from about 20 to 160 mg, even more preferably an amount of a natural extract containing OPC of about 20 mg;
- an amount of DHA ranging from 50 to 500 mg ; or a fish oil containing DHA in an amount ranging from 10 to 1000 mg, preferably from 50 to 500 mg, more preferably from about 100 to 300 mg, even more preferably an amount of a fish oil containing DHA of about 100 mg;
- an amount of Vitamin B1 or benfotiamin ranging from 0.1 to 10 mg, preferably from 0.5 to 5 mg, more preferably from about 1.1 to 2.2 mg, even more preferably comprises an amount of vitamin B1 or benfotiamin of about 1.1 mg;
- an amount of Vitamin B6 ranging from 0.1 to 10 mg, preferably from 0.5 to 5 mg, more preferably from about 1.4 to 2.8 mg, even more preferably comprises an amount of vitamin B6 of about 1.4 mg;
- an amount of chromium ranging from 5 to 500 µg, preferably from 10 to 200 µg, more preferably from 15 to 150 µg, even more preferably comprises an amount of chromium of about 20 µg; and/or
- an amount of magnesium ranging from 10 to 1000 mg, preferably from 50 to 500 mg, more preferably from 90 to 350 mg, even more preferably comprises an amount of magnesium of about 93.75 mg.

According to another embodiment, the composition is intended to be used to provide:
- a daily amount of OPC ranging from 1 to 200 mg/day, preferably from 5 to 150 mg/day, more preferably from about 10 to 112 mg/day, even more preferably an amount of OPC of about 13 mg/day; or a daily amount of a natural extract containing OPC ranging from 1.5 to 300 mg/day, preferably from 7.5 to 215 mg/day, more preferably from about 20 to 160 mg/day, even more preferably an amount of natural extract containing OPC of about 20 mg/day may be administered to a subject;
- a daily amount of DHA ranging from 100 to 250 mg per day; or a daily amount of a fish oil containing DHA ranging from 10 to 1000 mg/day, preferably from 50 to 500 mg/day, more preferably from about 100 to 300 mg/day, even more preferably an amount of fish oil containing DHA of about 100 mg/day may be administered to a subject;
- a daily amount of Vitamin B1 or benfotiamin ranging from 0.1 to 10 mg/day, preferably from 0.5 to 5 mg/day, more preferably from about 1.1 to 2.2 mg/day, even more preferably an amount of vitamin B1 or benfotiamin of about 1.1 mg/day may be administered to a subject;
- a daily amount of Vitamin B6 ranging from 0.1 to 10 mg/day, preferably from 0.5 to 5 mg/day, more preferably from about 1.4 to 2.8 mg/day, even more preferably an amount of vitamin B6 of about 1.4 mg/day may be administered to a subject;
- a daily amount of chromium ranging from 5 to 500 µg/day, preferably from 10 to 200 µg/day, more preferably from 15 to 150 µg/day, even more preferably an amount of chromium of about 20 µg/day may be administered to a subject; and/or
- a daily amount of magnesium ranging from 10 to 1000 mg/day, preferably from 50 to 500 mg/day, more preferably from 90 to 350 mg/day, even more preferably an amount of magnesium of about 93.75 mg/day may be administered to a subject.

According to an embodiment the composition further comprises excipients, such as for example coloring agents; flavoring agents; thickeners, such as, for example, glycerol monostearate; sweeteners; coating agents, such as, for example, beeswax, sunflower oil, refined colza oil, soya oil, peanut oil, sunflower lecithin, soya lecithin or fish gelatin; diluting agents, such as, for example, lactose, monohydrated lactose or starch; binding agents, such as, for example, povidone, pregelatinized starch, gums, saccharose, polyethylene glycol (PEG) 4000 or PEG 6000; disintegrating agents, such as, for example, microcrystalline cellulose or sodium carboxymethyl starch, such as, for example, sodium carboxymethyl starch type A; lubricant agents, such as, for example, magnesium stearate; flow agent, such as, for example, silica, colloidal anhydrous silica, etc....

Advantageously, the above-mentioned excipients are present in an amount ranging from 10 to 50% in weight to the total weight of the composition, preferably from 15 to 40% w/w, more preferably from 20 to 30% w/w, even more preferably in an amount of about 23.4% in weight to the total weight of the composition.

According to the invention, the composition of the invention is to be orally administered.

According to the invention, the composition may be in a solid form (pill, tablet, capsule, soft gelatin capsule, sugar-coated pill, orodispersing/orodisintegrating tablet, powder, effervescent tablet, etc...), in a liquid form (drinkable solution...), or in the form of a gel. According to a preferred embodiment, the composition is in a solid form, preferably is soft gelatin capsule.

According to an embodiment, the composition is formulated for sustained release thereof. In an embodiment, when formulated for sustained release thereof, the composition is in a solid form, preferably is a coated pill, a coated tablet, a soft gelatin tablet or a pill with modified release kinetic. According to an embodiment, the composition of the invention may be encapsulated, especially micro-encapsulated. Injection or ingestion of micro-encapsulated elements shall lead to the sustained release of the enzyme at the desired site, preventing any prior degradation of said element by the digestive apparel. Micro-encapsulation may be based on polymerization or reticulation.

According to an embodiment, the composition is packaged as a unitary dosage. Preferred unitary dosage is a soft gel capsule.

According to an embodiment, the composition of the present invention is for use in the ophthalmic field.

According to an embodiment, the composition of the invention is for reducing the risk of eye damage associated with diabetes, such as, for example, eye damages due to diabetic retinopathy and/or glaucoma. According to an embodiment, the composition of the present invention may also be used for preventing the deterioration of retinal blood vessels due to chronic hyperglycemia.

According to an embodiment, the composition of the invention is for inhibiting the development and/or progression of diabetic retinopathy and/or glaucoma.

According to an embodiment, the composition is for preventing diabetic retinopathy and/or glaucoma, and/or for reducing the risk of developing a diabetic retinopathy and/or glaucoma.

According to an embodiment, when the subject is already diagnosed with diabetic retinopathy, the composition of the invention is used for stabilizing, alleviating, and/or delaying the development of a diabetes-related eye condition such as for example diabetic retinopathy (proliferative and non-proliferative diabetic retinopathy) or glaucoma.

This invention also relates to a medicament comprising the composition of the invention. This invention also relates to a pharmaceutical composition comprising the composition of the invention in assocation with any pharmaceutically accepted excipient. This invention also relates to the medicament or pharmaceutical composition of the invention for use in the treatment of a diabetes-related eye condition such as for example diabetic retinopathy (proliferative and non-proliferative diabetic retinopathy) or glaucoma.

The present invention also relates to a method for reducing the risk of eye damage associated with diabetes, such as, for example, eye damages due to diabetic retinopathy in a subject in need thereof, said method comprising the administration of the composition of the invention. According to an embodiment, the method of the invention may also be for preventing the deterioration of retinal blood vessels due to chronic hyperglycemia.

According to an embodiment, the method of the invention is for inhibiting the development and/or progression of a diabetes-related eye condition such as for example diabetic retinopathy (proliferative and non-proliferative diabetic retinopathy) or glaucoma or oedema.

According to an embodiment, the method of the invention is useful for preventing a diabetes-related eye condition such as for example diabetic retinopathy (proliferative and non-proliferative diabetic retinopathy) or glaucoma and/or for reducing the risk of developing a diabetic retinopathy, in a subject in need thereof, by administration of a suitable amount of the composition of the invention.

In an embodiment, this invention relates to a method of treatment of diabetes-related eye condition such as for example diabetic retinopathy (proliferative and non-proliferative diabetic retinopathy) or glaucoma, including administering in a subject in need thereof, a therapeutic amount of the composition of the invention.

According to an embodiment, when the subject is already diagnosed with a diabetes-related eye condition, the method of the invention may be used for stabilizing, alleviating, delaying and/or reversing diabetic retinopathy.

In a preferred embodiment, the composition of the invention is administered to a subject in need thereof. According to an embodiment, the subject is an animal, preferably a mammal, more preferably a human.

According to an embodiment, the subject to whom the composition of the invention is administered is diagnosed with diabetes, such as, for example, type 1 or type 2 diabetes. According to another embodiment, the subject is diagnosed with diabetic retinopathy and/or glaucoma.

According to an embodiment, the subject to whom the composition of the invention is administered is at risk of developing diabetes or a diabetic retinopathy. According to an embodiment, the subject presents a genetic predisposition or a family antecedent with diabetes. According to another embodiment, the subject presents a non-genetic risk of developing diabetes. Examples of non-genetic risks of developing diabetes are obesity, such as, for example, a BMI (Body Mass Index) superior to 30; sedentary lifestyle; unhealthy eating habits, increased age, such as, for example, preferably more than 40, more than 50, more preferably more than 60 years old; high blood pressure and high cholesterol.

According to another embodiment, a unitary dosage of the composition of the invention is administered at least once a day, preferably once a day, during a recommended period of at least 1 to 3 weeks. Such treatment may be repeated one to three times. In severe pathologies, the recommended period of treatment may be indefinite, which means that the treatment may be a chronic treatment.

The present invention also relates to a veterinarian product, comprising the composition of the invention. In the embodiment, preferably, the subject is an animal, such as for example cat, dog, hamster, rabbit, mouse, gerbil, rat, Guinea pig.

According to an embodiment, the composition of the invention does not comprise an ingredient selected from the group comprising osmotine and fragments thereof.

According to an embodiment, the composition of the invention does not comprise an ingredient selected from the group comprising beta adrenergic antagonist, such as, for example, propranolol; anti-inflammatory agent, such as, for example, nonsteroidal anti-inflammatory drug; angiotensin-converting enzyme inhibitor; angiotensin receptor blocker; anabolic steroid; and dextromethorphan.

According to an embodiment, the composition of the invention does not comprise an ingredient selected from the group comprising Vitamin E, such as, for example, tocopherol and tocotrienol; Vitamin C and Vitamin B12.

According to an embodiment, the composition of the invention does not comprise an ingredient selected from the group comprising metallo-proteins, such as, for example, lactoferrin, transferrin, ovotransferrin, ceruloplasmin and metallo-thionein; and amino-acids.

According to an embodiment, the composition of the invention does not comprise a micronutrient selected from the group comprising coenzyme Q10, carnitin, taurin, inositol, alpha-lipoic acid and its reduced form (DHLA).

According to an embodiment, the composition of the invention does not comprise an ingredient selected from the group comprising vegetal oils selected from the group comprising colza oil, olive oil, grape seed oil and evening primrose oil; gingkolides; flowers or extracts thereof, such as for example Rose flower water distillate and Orange blossom flower distillate; macqui berry or an extract thereof; Açai fruit or an extract thereof and Jucara fruit or an extract thereof.

According to an embodiment, the composition of the invention does not comprise an ingredient selected from the group comprising polymethoxylated flavones and astaxanthin.

According to an embodiment, the composition of the invention does not comprise fucoidan.

According to an embodiment, the composition of the invention does not comprise a neovascular regulator selected from the group comprising genistein, daidzein, soy isolate (a specific source of genistein and/or daidzein), cartilage and chondroitin sulphate.

### BRIEF DESCRIPTION OF THE DRAWINGS

Figure 1 is a histogram showing the body weight regulation of Type-1 streptozotocin-induced diabetic Sprague-Dawley rats after 90 days of treatment.
Figure 2 is a histogram showing the glucose regulation of Type-1 streptozotocin-induced diabetic Sprague-Dawley rats after 90 days of treatment.
Figure 3 is a histogram showing GHb (glycated hemoglobin) regulation of Type-1 streptozotocin-induced diabetic Sprague-Dawley rats after 90 days of treatment.
Figure 4 is a histogram showing the AGE (advanced glycation end product) regulation of Type-1 streptozotocin-induced diabetic Sprague-Dawley rats after 90 days of treatment.
Figure 5 is a histogram showing the inhibition of diabetes-induced acellular capillary formation induced by treatment with OPC and DHA formulation. Values are means±SD. **p < 0.01, ***p < 0.001.
Figure 6 is a histogram and a curve showing the inhibition of 5-lipoxygenase (curve) by treatment with OPC and DHA formulation in diabetic animals in correlation with pericytes ghost count (histogram). Values are means±SD. **p < 0.01, ***p < 0.001.
Figure 7 is a graph showing inflammation inhibition following cyclooxygenase-2 (COX-2) and 5-lipoxygenase. Values are means±SD. **p < 0.01, ***p< 0.001.

### EXAMPLES

The present invention is further illustrated by the following examples. These examples are not meant to limit the scope of the invention.

### Example 1: Composition of the invention (per unitary dosage)

| | Ingredients | Quantity (mg) | Mg/unitary dosage |
|---|---|---|---|
| Active agents | Vitamin B 1 | 1,1 mg | 1,49 mg |
| | Vitamin B6 | 1,4 mg | 1,87 mg |
| | *Pinus pinaster* extract, rich in OPC | 20 mg | 20 mg |
| | Fish oil containing DHA (Epax® and Qualitysilver® labels) | 100 mg | 250 mg |
| | Magnesium | 93,75 mg | 155,47 mg |
| | Chromium | 20 µg | 100 µg |
| Excipients | Sunflower lecithin, sunflower oil, beeswax, glycerol monostearate, silica | | 131 mg |
| | | TOTAL | 560 mg |

### Example 2: Biological example

### Material and methods

### Experimental animal models: Streptozotocin-induced diabetic Rat model:

Sprague-Dawley rats (Harlan), weighing 75-100 g, were assigned to one of eight subgroups, based on non-diabetic, untreated diabetic and treated diabetic groups as indicated here-under. Sprague-Dawley rats were housed in a constant environment (room temperature (22.0±1.5)°C, room humidity (55±5)%) with a normal light-dark cycle (12-hour light, 12-hour dark). Diabetes was induced by intraperitoneal streptozotocin injection (75 mg/kg STZ, 10 mM citrate buffer, pH 4.5). Non-diabetic control animals received an equivalent dose of vehicle (citrate buffer at pH 4.6).

### Treatment of animals

To analyse the effect of the composition of the invention, animals are fed as followed:
Group G': Non-diabetic animals
Group G0: Diabetic animals (untreated)
Group G1: Diabetic animals fed with OPC
Group G2: Diabetic animals fed with DHA
Group G3: Diabetic animals fed with OPC + DHA
Group G4: Diabetic animals fed with OPC + DHA + Vit-B1/B6
Group G5: Diabetic animals fed with OPC + DHA + Chrome+ Magnesium
Group G6: Diabetic animals fed with the preferred composition of the invention of example 1 (OPC + DHA + Chrome+ Magnesium + Vit-B1/B6)

### Blood glucose concentration

Diabetic and non-diabetic groups were examined biweekly to follow blood glucose and the body weight was recorded. 50 µL of whole blood was collected in potassium-EDTA tubes (Becton Dickinson) for subsequent analysis of glycated hemoglobin (GHb). Four days after induction of diabetes, animals with high blood glucose concentrations (above 350 mg/dL for Sprague-Dawley rats,) (Glucometer Elite XL kit, Bayer) were considered diabetic.

### Retinal Carboxymethyl-Lysine measurement (AGE-product quantification)

Retinal samples were exposed to ultrasonic disruption in RIPA buffer. After tissue debris suppression by centrifugation, protein level of samples was measured using bicinchoninic acid (BCA) protein assay kit (Pierce). Sample (concentration: 50 µg/mL) or advanced glycation end product (AGE)-BSA standard (0 to 200 µM) diluted in 0.05% Tween 20 and 0.2% BSA in 75 mM PBS were placed in duplicate into 96-wells plate (Nunc C96 Maxisorp; eBioscience) coated with solid-phase antigen (1 µg/mL AGE-BSA). Anti-CML rabbit polyclonal antibody was added for 2 hours at room temperature and then washed in 0.2 mM KH₂PO₄, 1.4 mM K₂HPO₄, 3 mM NaCl, 0.45 µM sorbic acid potassium, and 0.01% Tween-20 buffer. Peroxidase-conjugated anti-rabbit IgG (Sigma-Aldrich) was added for 1 hour at room temperature and washed again. Colour reaction started by the addition of tetramethyl-benzine substrate (150 µL TMB; Sigma-Aldrich). Reaction was stopped after 30 minutes by the addition of 2 M sulfuric acid (50 µL). Absorbance was measured at 450 nm on a microplate reader (Biorad).

### Enzyme-Linked Immunosorbent Assay for inflammatory parameters

COX-2 enzymatic activity was measured using the COX Fluorescence Activity Assay Kit. Both assays are done following the manufacturer's instructions (Cayman Chemical Company, Ann Arbor, MI).

### ADPase enzyme histochemistry

Evaluation of the retinal vasculature is done following adenosine diphosphatase (ADPase) activity in whole embedded retina. After overnight fixation in 2% PFA in 0.1 M cacodylate buffer at 4°C, an incision was made 2 mm posterior to the ora serrata, and the anterior segment of the eye was removed. After the vitreous was removed from the eye cup, the retina was carefully separated from the RPE and choroid, washed in 0.1 M cacodylate buffer with 5% sucrose, and incubated for enzyme histochemical demonstration of ADPase activity. The ADPase-incubated retina was washed in 0.1 M cacodylate buffer with 5% sucrose. Radial cuts were made from the retinal periphery to points within 1 mm from the optic disc, to relax the retina before flat fixation in one-fourth strength Karnovsky's buffer for 72 hours. After 3 brief washes in 0.1 M cacodylate buffer with 5% sucrose, the fixed retinas were dehydrated in graded alcohols and flat embedded in resin (JB4; PolySciences Europe). Retinal microvascular density was quantified using Image J software.

### Quantitation of acellular capillaries and pericyte ghosts

After TUNEL-positive cells were counted, coverslips were soaked off, and the retinal vasculature then was stained with hematoxylin-periodic acid-Schiff. Acellular capillaries were counted in four to seven field areas in the mid retina. Acellular capillaries were identified as capillarysized vessel tubes having no nuclei anywhere along their length. Pericyte ghosts were estimated from the prevalence of spaces in the capillary from which pericytes had disappeared. Although counting the ghosts, at least 1000 capillary cells (endothelial cells and pericytes) were counted in five mid-retinal field areas. Ghosts on any acellular vessel were excluded.

### Reverse-Transcription PCR

Total RNA was isolated using (RNeasy kit; Qiagen). For first-strand cDNA synthesis, 2 µg RNA was incubated at 37°C, 50 minutes in diethyl pyrocarbonate (DEPC) water with 4 µL of first-strand buffer (5x), 1 µL dNTP mix (10 mM), 1 µL random primers, 1 µL RNase inhibitor (10 U/µL), 2 µL dithiothreitol (0.1 M), 1 µL M-MLV reverse transcriptase (Invitrogen). Primer sequences used were: β-actin forward, 5'-CAG-AAG-GAG-ATT-ACT-GCT-CTG-GCT-3'; β-actin reverse, 5'-GTG-AGG-GAC-TTC-CTG-TAA-CCA-CTT-3'; 5-lipoxygenase forward, 5'-ATG-GAT-GGA-GTG-GAA-CCC-CGG-3'; 5-lipoxygenase reverse, 5'-CTG-TAC-TTC-CTG-TTC-TAA-ACT-3'. PCR was performed with 2 µL reverse transcription products in 10 µL PCR buffer (5x) containing MgSO₄ (7.5 mM) and dNTP (10 mM), 1 µL DNA polymerase (2.5 U) (HotStar HiFidelity; Qiagen) and 1 µM of each forward and reverse primer in a total volume of 50 µL DEPC water per tube. Peltier thermal cycler (PTC-20035) is used and PCR cycles were carried out at 95°C for 5 minutes, 94°C for 15 seconds, 57°C for 1 minute, and 72°C for 1 minute, with a final extension at 72°C for 10 minutes. Reaction products were separated on a 1% agarose gel, and bands were visualized using ethidium bromide (0.1%). The gel was scanned with an imaging system (Bio-Rad).

### Results:

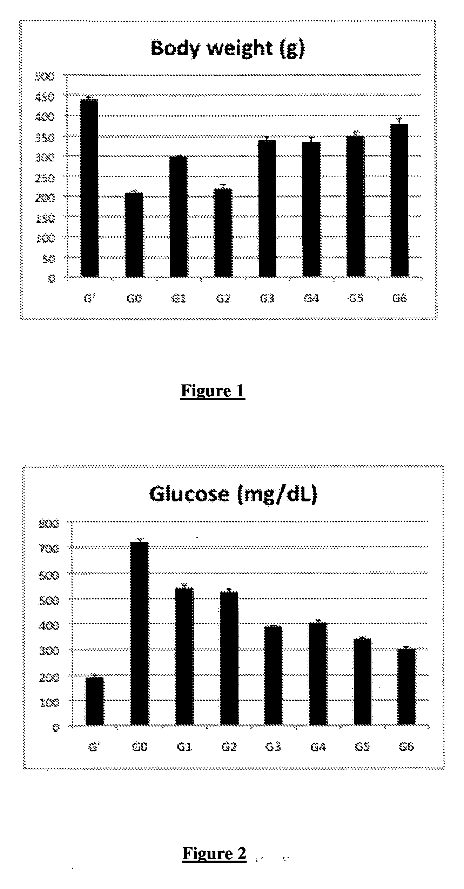

To study the efficiency of the composition of the invention, several animal models are used. One is constitutive type-I diabetic and three are inducible type-I diabetic models in mouse and rats. Results shown on figures 1 to 6 are for an inducible type-I diabetic rat model, but similar results were obtained with the three other models. Results shown on figures 1-4 are reported in the following table.

**Table 1: Type-1 streptozotocin-induced Diabetic Sprague-Dawley rats after 90 days of treatment.**

| Groups | n/groups | Body weight (g) | Glucose (mg/dL) | GHb (%) | AGE (AU/mg) |
|---|---|---|---|---|---|
| G' | 20 | 440 +/- 6 | 190 +/- 10 | 4.75 +/- 0.05 | 0.014 +/- 0.005 |
| **G0** | **20** | **210 +/- 5** | **720 +/**-**11** | **7.15 +/- 0.25** | **0.039 +/- 0.009** |
| G1 | 20 | 300 +/- 2 | 540 +/- 13 | 5.70 +/- 0.05 | 0.026 +/- 0.005 |
| G2 | 20 | 220+/-10 | 525 +/- 10 | 5.85 +/- 0.03 | 0.026 +/- 0.004 |
| **G3** | **20** | **340 +/- 8** | **390 +/- 2** | **5.01 +/- 0.05** | **0.021 +/- 0.007** |
| G4 | 20 | 335 +/-11 | 405 +/- 9 | 5.02 +/**-** 0.04 | 0.023 +/- 0.009 |
| **G5** | **20** | **350 +/-10** | **340 +/- 5** | **4.90 +/**- **0.02** | **0.020 +/- 0.004** |
| **G6** | **20** | **380 +/- 13** | **300 +/- 7** | **4.91 +/- 0.01** | **0.021 +/- 0.003** |

| | | | | | |
|---|---|---|---|---|---|
| Values are means +/- Standard error of the mean. GHb : glycated hemoglobin. AGE : advanced glycation end product. | | | | | |

An additive effect is observed with the association DHA-OPC. Moreover the addition of chromium, magnesium and/or Vitamine-B1/-B6, brought protective actions against glucose and vessel defection, respectively.

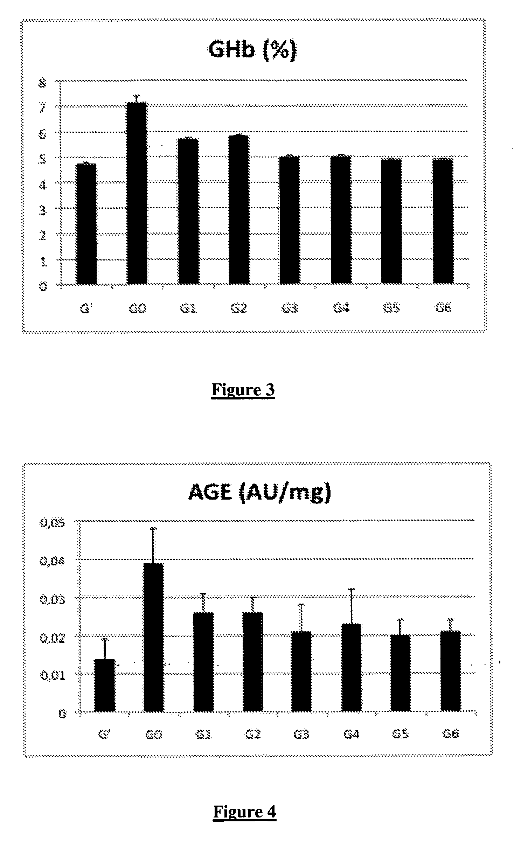

Taken together, (OPC + DHA) association cooperates to decrease the lost of body weight (figure 1), glucose (figure 2), Ghb (figure 3) and AGE (figure 4) in type-I diabetic models. Moreover, addition of mineral compounds (chromium, magnesium) that contribute to the regulation of glucose metabolism and Vitamine-B1/-B6 reinforced the effect of this initial compounds association in the favour of diabetes biomarkers (metabolite parameters) decreased.

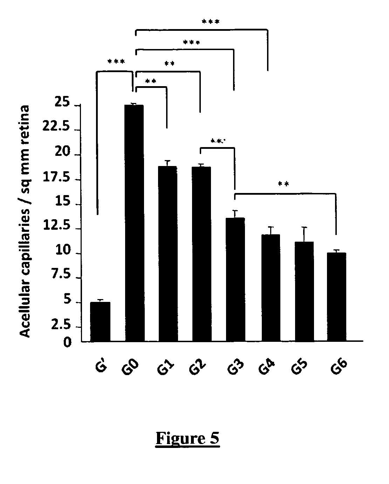

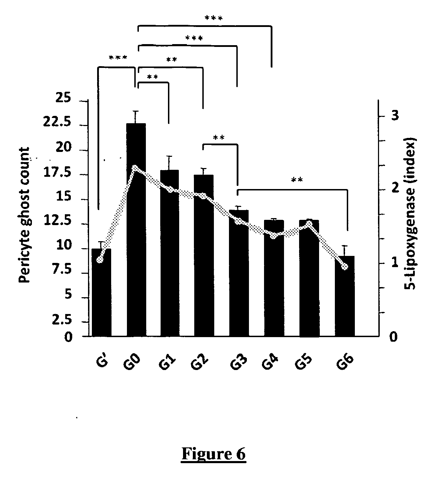

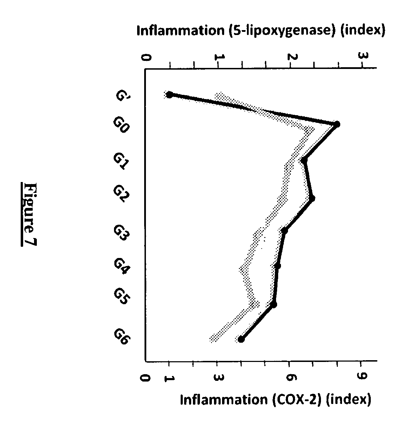

Compared to retina from non-diabetic animals, regional micro-vascular degeneration is clearly observed in diabetic individuals. Diabetic animals also harboured a significant increased in the number of degenerated acellular capillaries compared to untreated non-diabetic (figure 5). Capillary degeneration was significantly inhibited in diabetic animals receiving (OPC, DHA, OPC + DHA, OPC + DHA + Vitamine-B1/-B6 or the composition of example 1, containing OPC + DHA + chrome + Magnesium + Vitamine-B1/-B6) following additive or synergistic effect depending of groups, respectively (figure 5). Similarly, an increased degeneration of retinal pericytes was observed in diabetic animals compared to non-diabetic, but also a protection is obtained using previously described compounds association (figure 6). Taken together, these observations on the protection of retinal pericytes and acellular capillaries correlated with the inhibition of 5-Lipoxygenase by the compounds formula (figure 6). Figure 7 adds information about inflammation inhibition following cyclooxygenase-2 (COX-2) and 5-lipoxygenase.

The most important enzymes associated to inflammation induced by diabetes are both the cyclooxygenase-2 (COX-2) and the 5-lipoxygenase, which are followed to argue the anti-inflammatory effect of the formula. As redundantly shown in the figure 6 and figure 7, the 5-lipoxygenase is significantly diminished. Similarly to the 5-lipoxygenase, the cyclooxygenase-2 (COX-2), which is severally induced by diabetes more than 80-fold in diabetic patients, is drastically down-modulated by the formula (Figure 7). Both enzymes are implicated in the inflammatory response due to their implication in the regulation of protaglandins and leukotriens production. Taken together, down-modulation of the respectively cited enzymes activity decreased inflammation associated to this disease.

## Claims

1. A composition consisting of oligoproanthocyanidins (OPC), omega-3 fatty acid, Vitamin B1, Vitamin B6, chromium and magnesium, for use in preventing or stabilizing the development and/or the progression of a diabetes-related eye condition in a subject.

2. The composition for use according to claim **1**, wherein the OPC is natural, synthetic or hemisynthetic.

3. The composition for use according to anyone of claims **1** to **2**, wherein the OPC is natural and the composition comprises as OPC a OPC-containing natural extract, preferably a vegetal extract, more preferably an extract from a plant of the Pinacae family, even more preferably an extract from *Pinus pinaster* or *Pinus maritima*.

4. The composition for use according to anyone of claims **1** to **3**, wherein the omega-3 fatty acid is DHA, EPA or mixtures thereof.

5. The composition for use according to anyone of claims **1** to **4**, wherein the composition comprises a fish oil containing DHA, preferably a fish oil containing DHA having both the Epax® and the Qualitysilver® labels.

6. The composition for use according to anyone of claims **1** to **5**, wherein vitamin B1 is thiamin or benfotiamin, preferably thiamin.

7. The composition for use according to anyone of claims **1** to **6**, wherein vitamin B6 is pyridoxamine, pyridoxal, pyridoxine or mixtures thereof, preferably pyridoxamine.

8. The composition for use according to anyone of claims **1** to **7**, wherein chromium is present in the form of chromium picolinate, chromium chloride, chromium GTF®, chromium polynicotinate or mixtures thereof, preferably chromium picolinate.

9. The composition for use according to anyone of claims **1** to **8**, wherein magnesium is present in the form of magnesium gluconate, magnesium citrate, magnesium chloride, magnesium malate, magnesium orotate, magnesium from the sea, preferably magnesium gluconate.

10. The composition for use according to anyone of claims **1** to **9**, wherein the diabetes-related eye condition is a condition of the interior of the eye, preferably glaucoma or a retinal condition.

11. A functional food, a nutraceutical composition or a food or dietary supplement comprising a composition for use according to anyone of claims **1** to **10**.

12. The functional food, nutraceutical composition or food or dietary supplement according to claim **11**, comprising:
- 1 to 200 mg of OPC, or 1.5 to 300 mg of a natural extract containing OPC;
- 100 to 250 mg of DHA, or 10 to 1000 mg of a fish oil containing DHA;
- 0.1 to 10 mg of Vitamin B1;
- 0.1 to 10 mg of Vitamin B6;
- 5 to 500 µg of chromium; and
- 10 to 1000 mg of magnesium.

13. A medicament comprising a composition for use according to anyone of claims **1** to **10**.

14. A pharmaceutical composition comprising a composition for use according to anyone of claims **1** to **10**, in association with a pharmaceutically acceptable excipient.

15. A veterinarian product comprising the composition for use according to anyone of claims **1** to **10**.

## Patentansprüche

1. Zusammensetzung, bestehend aus Oligoproanthocyanidinen (OPC), Omega-3-Fettsäure, Vitamin B1, Vitamin B6, Chrom und Magnesium, zur Verwendung bei der Verhinderung oder der Stabilisierung der Entwicklung und/oder des Fortschreitens eines Diabetes-bezogenen Augenzustands in einem Subjekt.

2. Zusammensetzung zur Verwendung nach Anspruch **1**, wobei das OPC natürlich, synthetisch oder halbsynthetisch ist.

3. Zusammensetzung zur Verwendung nach einem der Ansprüche **1** bis **2**, wobei das OPC natürlich ist und die Zusammensetzung als OPC einen OPC-enthaltenden natürlichen Extrakt umfasst, vorzugsweise einen pflanzlichen Extrakt, insbesondere einen Extrakt der Familie Pinacae, noch bevorzugter einen Extrakt aus *Pinus pinaster* oder *Pinus maritima*.

4. Zusammensetzung zur Verwendung nach einem der Ansprüche **1** bis **3**, wobei die Omega-3-Fettsäure DHA, EPA oder Mischungen daraus ist.

5. Zusammensetzung zur Verwendung nach einem der Ansprüche **1** bis **4**, wobei die Zusammensetzung ein Fischöl, das DHA enthält, umfasst, vorzugsweise ein Fischöl, das DHA mit sowohl der Epax® - als auch der Qualitysilver®-Markierung enthält.

6. Zusammensetzung zur Verwendung nach einem der Ansprüche **1** bis **5**, wobei Vitamin B1 Thiamin oder Benfotiamin ist, vorzugsweise Thiamin.

7. Zusammensetzung zur Verwendung nach einem der Ansprüche **1** bis **6**, wobei Vitamin B6 Pyridoxamin, Pyridoxal, Pyridoxin oder Mischungen daraus ist, vorzugsweise Pyridoxamin.

8. Zusammensetzung zur Verwendung nach einem der Ansprüche **1** bis **7**, wobei Chrom in der Form von Chrompicolinat, Chromchlorid, Chrom-GTF®, Chrompolynicotinat oder Mischungen daraus, vorzugsweise Chrompicolinat vorhanden ist.

9. Zusammensetzung zur Verwendung nach einem der Ansprüche **1** bis **8**, wobei Magnesium in der Form von Magnesiumgluconat, Magnesiumcitrat, Magnesiumchlorid, Magnesiummalat, Magnesiumorotat, Magnesium aus dem Meer, vorzugsweise Magnesiumgluconat, anwesend ist.

10. Zusammensetzung zur Verwendung nach einem der Ansprüche **1** bis **9**, wobei der Diabetes-bezogene Augenzustand ein Zustand des Augeninneren ist, vorzugsweise Glaucom oder ein Netzhautzustand.

11. Funktionelles Nahrungsmittel, nutrazeutische Zusammensetzung oder Nahrungsmittel- oder Diätzusatz, umfassend eine Zusammensetzung zur Verwendung nach einem der Ansprüche **1** bis **10**.

12. Funktionelles Nahrungsmittel, nutrazeutische Zusammensetzung oder Nahrungsmittel- oder Diätzusatz nach Anspruch **11**, umfassend:
- 1 bis 200 mg OPC, oder 1,5 bis 300 mg eines natürlichen Extrakts, enthaltend OPC;
- 100 bis 250 mg DHA, oder 10 bis 1000 mg eines Fischöls, enthaltend DHA;
- 0,1 bis 10 mg Vitamin B1;
- 0,1 bis 10 mg Vitamin B6;
- 5 bis 500 µg Chrom; und
- 10 bis 1000 mg Magnesium.

13. Arzneimittel, umfassend eine Zusammensetzung zur Verwendung nach einem der Ansprüche **1** bis **10**.

14. Pharmazeutische Zusammensetzung, umfassend eine Verbindung zur Verwendung nach einem der Ansprüche **1** bis **10** in Verbindung mit einem pharmazeutisch akzeptablen Trägerstoff.

15. Tierärztliches Produkt, umfassend die Zusammensetzung zur Verwendung nach einem der Ansprüche **1** bis **10**.

## Revendications

1. Composition constitué d'oligo-proanthocyanidines (OPC), d'acide gras oméga-3, de vitamine B1, de vitamine B6, de chrome et de magnésium, pour son utilisation pour prévenir ou stabiliser le développement et/ou la progression d'une condition oculaire liée au diabète chez un sujet.

2. Composition pour son utilisation selon la revendication **1**, dans laquelle l'OPC est naturel, synthétique ou hémisynthétique.

3. Composition pour son utilisation selon l'une quelconque des revendications **1** à **2**, dans laquelle l'OPC est naturel et la composition comprend en tant qu'OPC un extrait naturel contenant de l' OPC, de préférence un extrait végétal, plus préférentiellement un extrait d'une plante de la famille des Pinacea, encore plus préférentiellement un extrait de *Pinus pinaster* ou de *Pinus maritima*.

4. Composition pour son utilisation selon l'une quelconque des revendications **1** à **3**, dans laquelle l'acide gras oméga-3 est le DHA, l'EPA ou leurs mélanges.

5. Composition pour son utilisation selon l'une quelconque des revendications **1** à **4**, dans laquelle la composition comprend une huile de poisson contenant du DHA, de préférence une huile de poisson contenant du DHA ayant à la fois le label Epax® et le label Qualitysilver®.

6. Composition pour son utilisation selon l'une quelconque des revendications **1** à **5**, dans laquelle la vitamine B1 est la thiamine ou la benfotiamine, de préférence la thiamine.

7. Composition pour son utilisation selon l'une quelconque des revendications **1** à **6**, dans laquelle la vitamine B6 est la pyridoxamine, le pyridoxal, la pyridoxine ou leurs mélanges, de préférence la pyridoxamine.

8. Composition pour son utilisation selon l'une quelconque des revendications **1** à **7**, dans laquelle le chrome est présent sous la forme de picolinate de chrome, de chlorure de chrome, de chrome GTF®, de polynicotinate de chrome ou de leurs mélanges, de préférence de picolinate de chrome.

9. Composition pour son utilisation selon l'une quelconque des revendications **1** à **8**, dans laquelle le magnésium est présent sous la forme de gluconate de magnésium, de citrate de magnésium, de chlorure de magnésium, de malate de magnésium, d'orotate de magnésium, de magnésium marin, de préférence de gluconate de magnésium.

10. Composition pour son utilisation selon l'une quelconque des revendications **1** à **9**, dans laquelle la condition oculaire liée au diabète est une condition de l'intérieur de l'oeil, de préférence un glaucome ou une condition rétinienne.

11. Alicament, composition nutraceutique ou supplément alimentaire ou diététique comprenant une composition pour son utilisation selon l'une quelconque des revendications **1** à **10**.

12. Alicament, composition nutraceutique ou supplément alimentaire ou diététique selon la revendication **11**, comprenant :
- 1 à 200 mg d'OPC, ou 1.5 à 300 mg d'un extrait naturel contenant de l'OPC ;
- 100 à 250 mg de DHA, ou 10 à 1000 mg d'une huile de poisson contenant du DHA;
- 0.1 à 10 mg de vitamine B1 ;
- 0.1 à 10 mg de vitamine B6 ;
- 5 à 500 µg de chrome ; et
- 10 à 1000 mg de magnésium.

13. Médicament comprenant une composition pour son utilisation selon l'une quelconque des revendications **1** à **10**.

14. Composition pharmaceutique comprenant une composition pour son utilisation selon l'une quelconque des revendications **1** à **10**, en association avec un excipient pharmaceutiquement acceptable.

15. Produit vétérinaire comprenant la composition pour son utilisation selon l'une quelconque des revendications **1** à **10**.
